# FASCICULE DE BREVET EUROPEEN

(11) **EP 0 910 424 B1**
(45) Date de publication et mention de la délivrance du brevet: **05.04.2000**
(21) Numéro de dépôt: 97931839.1
(22) Date de dépôt: 01.07.1997
(51) Int. Cl.: A61M 16/04

(54) **FILTRE RESPIRATOIRE JETABLE POUR SUJET TRACHEOTOMISE**
EINWEGATEMFILTER FÜR TRACHEOTOMIERTE PATIENTEN
DISPOSABLE RESPIRATORY FILTER FOR TRACHEOTOMIZED SUBJECT

(30) Priorité: 01.07.1996 FR 9608171
(43) Date de publication de la demande: 28.04.1999
(73) Titulaire: BEZICOT, Robert, F-92160 Antony (FR); Bezicot, Eric, 92160 Antony (FR); Voranger, Daniele, 92160 Antony (FR)
(72) Inventeur: BEZICOT, Robert, F-92160 Antony (FR); Bezicot, Eric, 92160 Antony (FR); Voranger, Daniele, 92160 Antony (FR)
(74) Mandataire: CABINET BONNET-THIRION
(86) Numéro de dépôt international: FR9701170
(87) Numéro de publication internationale: WO9800189

(56) Documents cités:
- EP-A- 0 370 962
- DE-A- 3 525 658
- GB-A- 2 202 823
- US-A- 4 622 698
- US-A- 5 306 233

## Description

L'invention se rapporte à un filtre à placer, pour un sujet trachéotomisé, en couverture de l'orifice chirurgical abouchant, à la base du cou, la trachée à l'extérieur, filtre comprenant un plastron en étoffe filtrante présentant une découpe en encolure avec une bordure qui se prolonge par au moins une bande d'attache propre à former, conjointement avec cette bordure, une boucle fermée autour du cou du sujet par réunion de moyens de jonction complémentaires.

De tels filtres sont par exemple décrits dans le document DE-A-3 525 658.

On sait que, lors d'interventions chirurgicales portant sur les voies respiratoires supérieures, au-dessus de la trachée, de sujets humains, par exemple pour des sarcomes du larynx, on pratique, à la base du cou du sujet, un orifice chirurgical abouchant la trachée directement à l'extérieur pour permettre au sujet des échanges respiratoires, inspiration et expiration, autonomes.

Ces échanges respiratoires ne bénéficient alors pas des fonctions de filtrage, tiédissement et saturation en vapeur d'eau qui s'effectuent par le passage de l'air inspiré par les narines, les fosses nasales et le pharynx.

On a proposé, pour pallier l'abolition de ces fonctions, des nez artificiels où la présence, sur le trajet de l'air de respiration, d'une masse spongieuse hygroscopique assure la triple fonction de filtrage, tiédissement et saturation en vapeur d'eau, ces deux dernières fonctions résultant, à l'expiration, de la condensation de l'humidité de l'air sortant des poumons et, à l'inspiration, de l'évaporation de l'humidité retenue par la masse spongieuse, et des échanges thermiques correspondants. Un tel nez est décrit, notamment, dans le document de brevet FR-A-2 683 150.

Cependant, en raison du poids propre d'un nez artificiel, et du contact entre la matière de ce nez et les tissus, il arrive que le port d'un tel nez artificiel soit mal toléré par certains sujets. En outre, dans la période post-opératoire, avant cicatrisation complète et stabilisation de l'orifice chirurgical, la mise en place d'un nez artificiel est indésirable.

Il est possible, moyennant certaines précautions relatives à l'environnement, température et hygrométrie, d'atténuer les inconvénients de l'arrivée directe dans la trachée d'air à température et hygrométrie non ajustées. Mais la fonction de filtrage reste nécessaire et essentielle, sauf à maintenir le sujet en salle blanche. On observera que le filtrage nécessaire implique l'arrêt de corps étrangers relativement volumineux, tels que des parties de vêtements.

Dans les premiers temps de la période post-opératoire, la fonction de filtrage est assurée par des pansements agencés de façon appropriée pour permettre le passage de l'air tout en protégeant les tissus exposés. Mais ces pansements ne peuvent qu'être provisoires. Succédant aux pansements spécifiques, les dispositifs doivent être légers, faciles à mettre en place, efficaces tant dans leur rôle de filtre que dans leur rôle de protection contre l'intrusion de corps étrangers, et suffisamment stériles.

Il va de soi que l'efficacité du résultat attendu du filtrage suppose que le filtre plastron soit changé fréquemment et lavé régulièrement et avec soin. Par ailleurs, le coût de ces filtres n'est pas négligeable, en raison de leur structure, qui impose de nombreuses opérations de fabrication, et du stock nécessaire pour assurer un change régulier. En outre, la régularité du change et du lavage peut être atteinte par erreur ou négligence, entraînant des risques sérieux.

Les inventeurs ont pris conscience que l'hygiène souhaitable ne serait obtenue que si le filtre, stérile à l'état neuf, ne pouvait être réutilisé et soit jeté après un usage unique. Cela impliquait, d'une part, que le prix de revient soit faible (matière et processus de fabrication) pour que le sujet ne soit pas tenté de le réutiliser, et d'autre part, et surtout, que le filtre ne soit plus susceptible d'être réutilisé après un usage unique. Ce dernier résultat est obtenu en mettant en oeuvre des moyens de jonction opérant une seule fois, de sorte qu'il ne soit plus possible, après que le plastron a été retiré, de le fixer à nouveau autour du cou du sujet. On observera que les deux résultats, prix réduit et difficulté de réutilisation sont concourants pour dissuader le sujet de tenter de réutiliser le filtre. On observera par ailleurs que, d'une façon générale, les articles à usage unique, tels les mouchoirs en papier, sont pratiquement mis hors service par l'usage qui en est fait, tandis que les filtres respiratoires ne manifestent pas que leur premier usage les rend impropres à la protection.

Ainsi, l'invention consiste en un filtre à placer, pour un sujet trachéotomisé, en couverture de l'orifice chirurgical abouchant, à la base du cou, la trachée à l'extérieur selon la revendication 1.

La feuille de non-tissé réunit les avantages d'un faible coût, d'une réalisation facile par découpe à la presse, d'une aptitude à l'ajustement de la porosité avec des pores par nature de faibles dimensions, et d'une cohésion intrinsèque limitée, propice à une mise hors service des moyens de jonction après séparation.

Grâce à la baleine étendue radialement, on évite l'obstruction de l'orifice chirurgical sous l'effet du souffle.

En disposition préférée, le plastron comporte, sur une face destinée à venir au contact du torse du sujet, une plage adhésive en forme de bande adhésive disposée transversalement sensiblement au ras de l'encolure. Cette plage adhésive vient se fixer sur le cou du sujet, au-dessus de l'orifice chirurgical. Elle assure ainsi que l'étoffe filtrante est maintenue étalée au devant de cet orifice chirurgical, malgré les mouvements du sujet et le frottement des vêtements. En outre, l'ablation au moins partielle du larynx résultant de la trachéotomie provoque une dépression à la base du cou, autour de l'orifice chirurgical. Le maintien de l'étoffe filtrante sur le cou au-dessus de cet orifice chirurgical évite qu'il se forme un espace entre le plastron et le cou par où pourrait passer de l'air non filtré.

Egalement, on préfère que le plastron comporte, au plus loin de l'encolure, deux plages adhésives aptes à maintenir le plastron à sa partie inférieure, contre la peau, pour parfaire l'étalement de ce plastron sur le torse.

Il est prévu que le filtre est mis à la disposition du sujet à l'état stérile, enfermé dans une poche étanche scellée.

Des caractéristiques secondaires et des avantages de l'invention ressortiront d'ailleurs de la description qui va suivre, à titre d'exemple, en référence aux dessins annexés dans lesquels :
la figure 1 représente un filtre selon l'invention, vu de l'envers ;
la figure 2 représente le filtre de la figure 1, à l'endroit, avec les bandes d'attache réunies ;
la figure 3 représente le filtre mis en place sur un sujet.

Selon l'invention et représenté sur les figures, un filtre respiratoire pour sujet trachéotomisé 1 dans son ensemble se présente comme une feuille d'étoffe non tissée découpée en forme de plastron, avec une échancrure 2 formant encolure, dont la bordure se prolonge par des bandes d'attache 3 et 3', droites, et d'une pièce avec le reste du plastron.

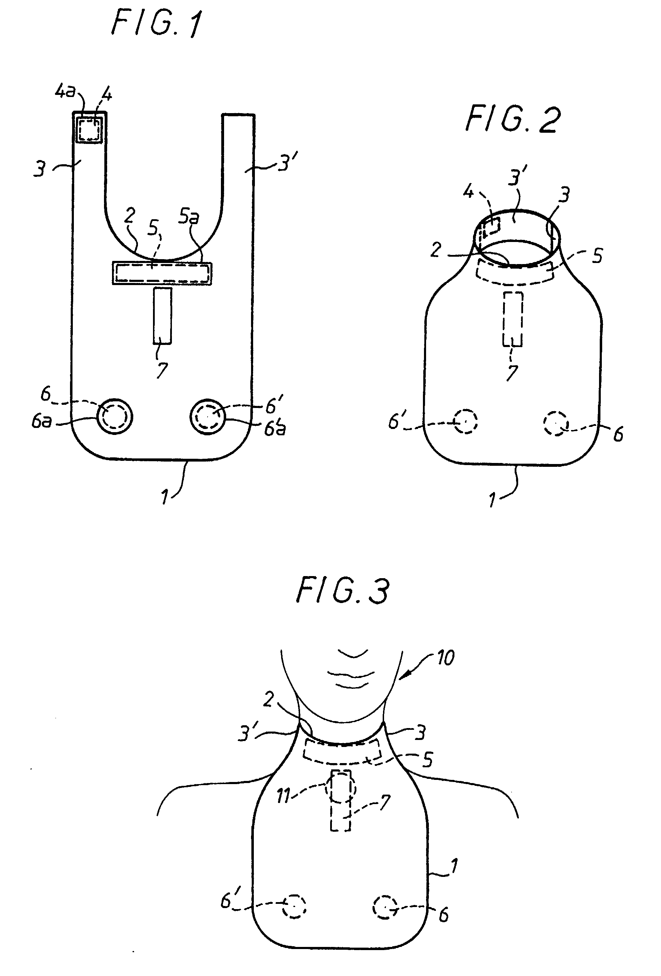

Sur la face de la feuille qui constitue le plastron 1, destinée à porter sur le torse du sujet, qui sera dite face envers, et qui est visible figure 1, la bande 3 (à gauche figure 1) comporte en extrémité une plage 4 d'adhésif de pression, recouverte, comme il est usuel, d'une feuille de protection 4a. Cette plage adhésive 4, comme représenté figure 2, permet, en coopération avec la face endroit de la bande d'attache 3', de former conjointement avec la bordure de l'encolure 2 une boucle fermée entourant le cou du sujet, comme on le voit sur la figure 3. La plage adhésive 4 de la bande 3 et la face endroit de la bande 3' constituent moyens de jonction complémentaires. L'adhésif qui constitue la plage 4 est choisi de consistance telle que la jonction soit résistante aux efforts de traction résultant des mouvements du sujet, et que la séparation ultérieure des bandes 3 et 3' entraîne l'impossibilité pratique de rétablir la jonction, soit que l'une des bandes soit déchirée, soit que la face externe de l'adhésif ait retenu des débris de non-tissé arrachés à la face endroit de la bande 3', et n'ait plus de pouvoir d'adhésion.

La face envers du plastron 1 porte, symétriquement par rapport à son milieu en direction radiale de l'encolure (ce milieu prenant une direction verticale lorsque le filtre est porté par un sujet comme représenté figure 3), une plage adhésive 5 en forme de bande disposée transversalement sensiblement au ras de l'encolure, recouverte d'une feuille de protection 5a. Egalement, au plus loin de l'encolure, cette face envers du plastron porte deux plages adhésives 6, 6', symétriquement par rapport à son milieu. Ces plages 6, 6' sont recouvertes de feuilles de protection 6a, 6'a.

Par ailleurs, une baleine 7 est collée sur la face envers du plastron, orientée suivant le milieu de celui-ci, avec une extrémité proche de l'encolure sensiblement à hauteur du bas de la plage adhésive 5. On précisera plus loin ces dispositions et leurs rôles.

On notera toutefois que l'adhésif utilisé pour les plages 5, 6, 6', doit être physiologiquement compatible avec la peau, tandis que celui qui constitue la plage 4 ne répond pas nécessairement à cet impératif. Mais il est possible de choisir un adhésif qui satisfait aux deux impératifs.

Cette baleine 7 est constituée d'une bande de matière polymère semi-rigide, telle qu'elle puisse s'adapter à la courbure du sternum, mais offre une résistance efficace au froissement.

Comme on le voit figures 2 et 3, pour joindre les deux bandes d'attache, la bande 3' est passée par-dessus l'épaule droite d'un sujet 10 (dont on a seulement esquissé les lignes du cou, du menton et des épaules), puis la bande 3, passée par-dessus l'épaule gauche, est appliquée sur la bande 3' de façon que la plage adhésive 4 vienne se coller sur cette bande 3' (face endroit), le cou du sujet 10 étant entouré étroitement.

Comme représenté figure 3, le sujet trachéotomisé 10 présente, à la base du cou, un orifice chirurgical 11 qui abouche la trachée à l'extérieur. Le plastron vient se placer de sorte que son milieu passe devant l'orifice chirurgical, et que la plage 5 soit disposée symétriquement par rapport à cet orifice et au-dessus de celui-ci. L'emplacement de la baleine 7 sur le plastron 1 a été réglé de telle façon qu'elle se place en pont sur l'orifice chirurgical 11. Ainsi la raideur de la baleine 7 et le collage de la plage adhésive 5 assurent conjointement que le plastron, à l'emplacement en regard de l'orifice chirurgical, couvre cet orifice sans, ni venir pénétrer dans cet orifice, ni laisser passer de l'air directement vers l'orifice sans traverser le non-tissé, ni former des plis qui créeraient des superpositions d'épaisseurs d'étoffe filtrante, augmentant les pertes de charge dans le filtre.

Les plages 6, 6' coopèrent au maintien du plastron 1 sur le sujet 10, en empêchant que le bas de ce plastron ne soit relevé par suite de mouvements du sujet combinés au frottement de vêtements placés par-dessus.

La mise à la disposition des usagers, trachéotomisés, se fera après stérilisation des filtres-plastrons par un procédé approprié, et introduction de chaque filtre dans une poche étanche qui sera ensuite scellée.

Notamment, la plage adhésive 4 pourrait être placée indifféremment sur l'une et l'autre des bandes d'attache 3, 3', et sur la face endroit aussi bien que la face envers, la disposition sur la face envers présentant toutefois l'avantage de permettre l'enduction de l'ensemble des plages 4, 5, 6, 6' en une seule opération.

## Revendications

1. Filtre à placer, pour un sujet trachéotomisé (10), en couverture de l'orifice chirurgical (11) abouchant, à la base du cou, la trachée à l'extérieur, filtre comprenant un plastron (1) en étoffe filtrante présentant une découpe en encolure (2) avec une bordure qui se prolonge par au moins une bande d'attache (3, 3') propre à former, conjointement avec cette bordure, une boucle fermée autour du cou du sujet (10) par réunion de moyen de jonction complémentaires (4, 3'), l'étoffe filtrante étant une feuille en non-tissé, le filtre comportant deux bandes d'attache (3, 3'), les moyens de jonction (4, 3') étant situés sur l'une et l'autre bande d'attache lesquelles (3,31) sont d'une pièce avec le plastron (1), l'un au moins des moyens de jonction (4) étant constitué par une couche d'adhésif de pression à usage unique, et le plastron (1) comportant en son milieu une baleine (7) étendue radialement par rapport à l'encolure (2), avec une extrémité proche de celle-ci telle qu'elle se place en pont sur l'orifice chirurgical (11) ladite baleine étant constituée d'une bande de matière polymère semi-rigide, telle qu'elle puisse s'adapter à la courbure du sternum, mais offre une résistance efficace au froissement.

2. Filtre selon la revendication 1, caractérisé en ce que le plastron comporte, sur une face destinée à venir au contact du torse du sujet, une plage adhésive (5) en forme de bande disposée transversalement sensiblement au ras de l'encolure (2).

3. Filtre selon la revendication 2, caractérisé en ce que le plastron comporte, au plus loin de l'encolure (2), deux plages adhésives (6, 6') aptes à maintenir le plastron (1), à sa partie inférieure, contre la peau du torse du sujet.

4. Filtre selon l'une quelconque des revendications 1 à 3, caractérisé en ce qu'il est mis à la disposition du sujet (10) à l'état stérile enfermé dans une poche étanche scellée.

## Patentansprüche

1. Filter zum Anbringen bei einer Person (10) mit Luftröhrenschnitt unter Abdeckung der chirurgischen Öffnung (11), welche an der Basis des Halses mit der Luftröhre zusammentrifft, nach außen, welcher Filter einen Brustteil (1) aus Filtermaterial aufweist, der einen Halsausschnitt (2) mit einem Randbereich darbietet, der sich durch wenigstens ein Befestigungsband (3,3') verlängert, das geeignet ist, in Verbindung mit dem Randbereich, eine Schlinge zu bilden, die um den Hals der Person (10) durch Vereinigung von komplementären Verbindungsmitteln (4,3') schließbar ist, wobei das Filtermaterial ein nicht gewebtes Blatt ist; der Filter zwei Befestigungsbänder (3,3') aufweist; die Verbindungsmittel (4,3') an dem einen und an dem anderen Befestigungsband sitzen und diese (3,3') ein Stück mit dem Brustteil (1) bilden; wenigstens eines (4) der Verbindungsmittel durch eine Schicht eines Druckklebers von nur einmaliger Verwendbarkeit gebildet ist; der Brustteil (1) in einem mittleren Bereich ein Stäbchen (7) aufweist, welches sich um den Halsausschnitt (2) radial erstreckt und dessen dem Halsausschnitt näher liegendes Ende sich wie eine Brücke über die chirurgische Öffnung (11) erstreckt; wobei besagtes Stäbchen von einem Band aus halbsteifem Polymermaterial gebildet ist, so daß es sich der Krümmung des Brustbeins anpassen kann, jedoch einen wirksamen Widerstand gegen Knittern darbietet.

2. Filter nach Anspruch 1, dadurch gekennzeichnet, daß der Brustteil auf einer Fläche, die dazu bestimmt ist, in Kontakt mit dem Rumpf der Person zu kommen, einen klebenden Bereich (5) in Bandform aufweist, der quer und im wesentlichen in Höhe des Halsausschnittes (2) angeordnet ist.

3. Filter nach Anspruch 2, dadurch gekennzeichnet, daß der Brustteil weiter entfernt von dem Halsausschnitt (2) zwei klebende Bereiche (6,6') aufweist, die geeignet sind, den Brustteil (1) in seinem unteren Bereich gegen die Haut des Rumpfes der Person zu halten.

4. Filter nach einem der Ansprüche 1 bis 3, dadurch gekennzeichnet, daß er zur Disposition der Person (10) in einem sterilen Zustand gehalten ist, eingeschlossen in einer dicht versiegelten Hülle.

## Claims

1. Filter for a tracheotomy patient (10), to be positioned so as to cover the surgical orifice (11) at the base of the neck connecting the trachea to the outside, said filter comprising a breastplate (1) of filter fabric having a neck cut-away (2) with an edge which is extended by at least one fastening strip (3, 3') capable of forming, together with said edge, a loop closed about the neck of the patient (10) through the connection of complementary joining means (4, 3'), the filter fabric being a nonwoven sheet, the filter comprising two fastening strips (3, 3'), the joining means (4, 3') being located respectively on the two fastening strips, which (3, 31) are in one piece with the breastplate (1), one at least of the joining means (4) consisting of a layer of single-use pressure-sensitive adhesive, and the breastplate (1) comprising in the centre thereof a stiffener (7) extending radially with respect to the neck cut-away (2), with one end close thereto such that it bridges the surgical orifice (11), said stiffener consisting of a strip of semirigid polymeric material, such that it may conform to the curvature of the sternum but provide effective resistance to creasing.

2. A filter according to claim 1, characterised in that the breastplate comprises, on a surface intended to come into contact with the torso of the patient, an adhesive area (5) in the form of a strip disposed substantially transversely level with the neck cutaway (2).

3. A filter according to claim 2, characterised in that the breastplate comprises, at points furthest from the neck cut-away (2), two adhesive areas (6, 6') capable of holding the breastplate (1), at the lower part thereof, against the skin of the patient's torso.

4. A filter according to any one of claims 1 to 3, characterised in that it is made available to the patient (10) in a sterile condition and enclosed in a sealed impermeable pouch.
